Europäisches Patentamt

European Patent Office

Office européen des brevets

(11) Publication number: **0 253 464 B1**

(12) **EUROPEAN PATENT SPECIFICATION**

(45) Date of publication of patent specification: **27.05.92**  (51) Int. Cl.5: **G01N 33/543**

(21) Application number: **87302403.8**

(22) Date of filing: **20.03.87**

(54) **Methods for providing internal references for use in analyte receptor assays.**

(30) Priority: **21.03.86 US 842611**

(43) Date of publication of application:
**20.01.88 Bulletin 88/03**

(45) Publication of the grant of the patent:
**27.05.92 Bulletin 92/22**

(84) Designated Contracting States:
**AT BE CH DE ES FR GB GR IT LI LU NL SE**

(56) References cited:
**EP-A- 0 171 150      EP-A- 0 186 100**
**EP-A- 0 200 381      EP-A- 0 203 238**
**US-A- 4 540 659      US-A- 4 632 901**

**PATENT ABSTRACTS OF JAPAN, vol. 8, no.
105 (P-274)[1542], 17 May 1984**

(73) Proprietor: **HYBRITECH INCORPORATED**
**11095 Torreyana Road**
**San Diego California 92126(US)**

(72) Inventor: **Valkirs, Gunars Edward**
**2893 Paseo del Sol**
**Encinitas, CA 92025(US)**
Inventor: **Anderson, Richard Ray**
**634 Hollyridge Drive**
**Encinitas, CA 92024(US)**

(74) Representative: **Tapping, Kenneth George et
al**
**Erl Wood Manor**
**Windlesham Surrey, GU20 6PH(GB)**

Rank Xerox (UK) Business Services

**Description**

The present invention relates generally to methods for performing assays; more particularly, it relates to methods for providing internal references for use in analyte-receptor assays.

This application is related to the subject matter of U.S. Patent No. 4,632,901. This application also relates to the subject matter of A. Rubenstein, EPO Patent Application EP-A-0200381.

Analyte-receptor assay procedures, particularly immunoassays, are useful for the in vitro determination of the presence and concentration in serum or other body fluids of analytes associated with diseases and other physiological conditions of clinical significance. One type of assay, the immunometric assay, provides an especially sensitive method for the quantitation of target analytes. Other analyte-receptor assays include assays involving nucleic acid probe technology.

Analyte-receptor assays generally involve the formation of a complex between a receptor, such as an antibody, bound to a solid phase, and a target analyte, for example an antigen, present in a sample. A receptor conjugate, such as a labeled anti-analyte antibody, is permitted to bind with the target analyte which has complexed with the receptor bound to the solid phase forming, for example, an antibody:analyte:antibody "sandwich". Alternatively, pre-complexed receptor conjugate and target analyte may be allowed to bind with the receptor immobilized on the solid phase. Immunometric assays in the past have relied on polyclonal antibody preparations; however, recent improvements in such assays include the use of monoclonal antibodies. U.S. Patent No. 4,376,110 describes two-site immunometric assays using pairs of monoclonal antibodies, one bound to a solid phase and the other labeled, for example with an enzyme, radioactive isotope or fluorescent material, to permit detection.

Receptors utilized in analyte-receptor assays typically are immobilized on a solid phase such as a filter or membrane. Alternatively, the receptors may be bound to microspheres entrapped within a porous matrix. Techniques for binding receptors to a solid phase are well-known in the art. For example, antibodies may be bound to polysaccharide polymers using the process described in U.S. Patent No. 3,645,090.

Receptor conjugates, utilized in analyte-receptor assays to bind with target analytes which are immobilized on a solid phase, are often labeled with an enzyme, such as alkaline phosphatase or horseradish peroxidase. When exposed to a substrate, such as indoxyl phosphate or tetramethyl benzidine, a product is formed generating a signal such as blue color development. The signal generated allows detection of the presence of enzyme-labeled receptor on the solid phase. The signal generated is related to the amount of bound, enzyme-labeled receptor which, in turn, is related to the amount of bound target analyte. These enzyme-labeled receptors complex with target analytes upon incubation to form analyte-labeled receptor complexes in a manner which is time, temperature and concentration dependent. Additionally, enzyme-substrate turnover exhibits time, temperature and concentration dependence. Enzyme-substrate systems used to generate measurable responses in analyte-receptor assays are, therefore, sensitive to effects such as fluctuations in temperature, incubation time and reagent concentration.

In addition to the foregoing limitations inherent in present analyte-receptor assays, such as immunometric assays, it is inconvenient and may not be possible to compare results between assays performed on different solid phases if controls or references are performed with each assay to establish separately the extent of such effects on the assay and to correct for them. Further, controls or references are necessary to validate or confirm that all the procedural steps of the assay were performed correctly with the test sample. To verify that the assay was performed properly when using a qualitative protocol, both a positive and a negative control are necessary for one unknown test sample. Previously, such verification has been provided by separate assays as controls, for example, using as a positive control a sample containing known levels of the analyte being assayed. To obtain quantitative results the number of controls, for example, calibrators, necessary to establish the dependence of the assay signal as a function of analyte concentration is dependent on the assay system. In the simplest case where the signal is a linear function of the analyte concentration, two calibration points are required for quantitation.

Problems with such separately run calibrators and controls include the occurrence of false positives in which a signal is generated due to a non-specific reaction of the sample components with the assay reagents. This produces a signal indicative of a positive result. Additionally, variations in assay parameters, such as, for example, incubation time for binding of reference receptor and sample analyte to receptor conjugate, temperature and labeled receptor concentration, affect the quantitation of unknown sample relative to the separately calibrated assay response.

The use of internal references of calibrators in analyte-receptor assays, particularly immunoassays, is described by Litman et. al., Clin. Chem, 29 1958 (1983) and U.S. Patent No. 4,540,659. Such internal calibrators are established by relating a specific reference signal with a particular sample analyte concentration. Although it is possible to establish a one-for-one correspondence between reference signal and analyte concentration, over an appropriate range of analyte concentration, the correspondence is variable, having a variable dependence on assay conditions. This is attributable, for example, to the non-linear dependence of the correspondence between reference signal and analyte concentration on assay parameters such as conjugate incubation time. Accordingly, a limitation inherent in such internal calibrators is that normal variations from specific assay conditions, such as time, temperature and reagent concentration, may invalidate the previously established relationship between internal calibrator and analyte concentration and, therefore, the determination of analyte concentration in unknown samples would be variable and imprecise.

A further limitation inherent in the internal calibrators described above is that the reference receptor must bind to a site on the labeled species, i.e., labeled receptor, other than the site for binding of analyte. Changes in the specific binding properties of the labeled species, therefore, may not be reflected by such internal calibrators.

Accordingly, a need exists for internal references for use in analyte-receptor assays which permit the determination of unknown analyte concentrations substantially independent of normal variations in assay conditions such as time, temperature and reagent concentrations, and further, are substantially independent of non-specific interfering substances in the sample. Further, there exists a need for internal references which reflect changes in specific binding properties of labeled receptors used in such assays. The present invention meets these needs by providing a method for the incorporation of such internal references in analyte-receptor assays for use in the qualitative and/or quantitative determination of analytes.

EP-A-171150 discloses an internal quality control or reference system for solid phase immuno assays using as a reference the analyte of interest bound to a solid phase, optionally via a receptor.

EP-A-203238 provides an assay system and apparatus for detecting ligands such as morphine in body fluids.

In particular, the present invention provides a method for providing internal references for use in a sequential analyte-receptor assay for the determination of a target analyte in a sample, the assay employing a signal producing system and an analyte receptor capable of binding the target analyte in an amount directly proportional to the amount of the target analyte in the sample, which comprises:

binding to a solid phase, at a discrete reference zone separate from a discrete test zone to which the analyte receptor is bound, a reference receptor selected to bind with an analyte receptor conjugate which is capable of complexation with the target analyte such that the rates of binding of the analyte receptor conjugate to the reference receptor and to the target analyte are directly proportional to the amounts of the reference receptor and the target analyte bound to the solid phase; the rate constants are substantially equivalent; and

whereby signal development at the reference zone and the test zone is directly proportional to the amount of the analyte receptor conjugate bound to the reference and test zones.

The following terms are used in the description and are as defined:

Analyte: A substance in a body fluid and a member of an analyte-receptor binding pair.

Antibody: A protein capable of complexing with an antigen.

Antigen: A substance which binds specifically to an antibody.

Anti-antibody Antibody: Antibody which complexes with the constant region of an antibody molecule.

Anti-idiotype Antibody: Antibody which complexes with the variable region of an antibody molecule.

Conjugate: The complex between a receptor or ligand and a label. In the present invention, two conjugates are described: 1) an analyte receptor conjugate comprising a receptor, such as an analyte receptor, and a label; and 2) a ligand conjugate comprising a ligand, and a label; alternatively, ligand conjugate may comprise a label.

First-Order Rate Constant: The binding of a target analyte to an analyte receptor, in a biomolecular reaction, is at a rate determined by the rate equation:

$$\frac{d[\text{analyte receptor:analyte}]}{dt} = K[\text{analyte receptor}] [\text{analyte}]$$

where K is the second-order rate constant for the binding of the analyte receptor and the target analyte. Provided that the concentration of the analyte receptor is in sufficient excess over the concentration of the target analyte such that it is effectively a constant during the course of the binding reaction in an assay, the rate of binding is directly proportional to the concentration of the target analyte and the first-order rate constant is K [analyte receptor]. In such a case, the first-order rate constant may be varied by selection of the concentration of analyte receptor. All such analyte-receptor binding reactions are subject to the same principles.

Hapten: A small separate part of an antigen capable of reacting specifically with an antibody, but incapable of stimulating antibody production except in combination with a carrier protein molecule.

Internal Reference: A means for calibrating the time, temperature and concentration dependence of the signal produced by an analyte-receptor assay system, independent of the unknown analyte concentration of the sample being assayed.

Label A substance capable of generating a signal.

Ligand: A substance, such as a protein, enzyme, hapten or oligonucleotide which by itself, or complexed with a label as in ligand conjugate, binds specifically to a receptor, excluding the target analyte and components of the sample being assayed.

Receptor: One member of an analyte-receptor or ligand conjugate receptor binding pair, e.g., antigen or antibody, enzyme receptor, hormone receptor.

Reference Receptor: A receptor which is capable of functioning as an internal reference in an assay by binding with a conjugate.

Reference Zone: A discrete zone on a solid phase to which reference receptor is bound.

Signal: A response generated at the reference and test zones, detectable by visual or instrumental means.

Signal Producing System: A system, preferably enzymatic, for generating a detectable signal at the reference and test zones whereby signal development is in a linear relationship with the amount of conjugate bound to such discrete zones.

Test Zone: A discrete zone on a solid phase to which an analyte-receptor capable of binding with a target antigen is bound.

In accordance with the present invention, a method is provided for incorporating internal references in analyte-receptor assays, employing a signal producing system, for the determination of at least one target analyte in a sample.

In the context of the present invention, the term "analyte-receptor assay" refers to an assay for an analyte which is accomplished by the formation of a complex between the analyte and another substance capable of specific interaction with the analyte, i.e., a receptor. The term "analyte," encompasses, for example, antigens, haptens, antibodies, deoxyribonucleic acid (DNA), ribonucleic acid (RNA), hormones, metabolites, and other naturally occurring substances of diagnostic interest having a specific binding partner associated with it, i.e., the receptor of the analyte-receptor assay.

Accordingly, the method provided by the present invention comprises binding a suitable reference receptor to a solid phase in a discrete reference zone. Such a reference zone is separate from a discrete test zone on the solid phase to which is bound an analyte receptor capable of binding the target analyte during an assay in an amount directly proportional to the amount of target analyte in the sample. The analyte receptor may be, for example, an antibody or other substance which selectively binds the analyte. For example, in an assay for allergy specific IgE, the analyte receptor may be an allergen. The reference receptor is selected to bind with an analyte receptor conjugate capable of complexation with the target analyte. Preferably, the reference receptor is selected from the target analyte, anti-enzyme antibody, anti-hapten antibody, anti-antibody antibody and anti-idiotype antibody. One will note, however, that the binding of analyte receptor conjugate to the reference receptor at the reference zone is independent of the binding of analyte receptor conjugate to the target analyte.

The method of the present invention is predicated, at least in part, upon the selection of a reference receptor such that the rates of binding of the analyte receptor conjugate to the reference receptor and to the target analyte are directly proportional to the amounts of the reference receptor and target analyte bound via the analyte receptor to the solid phase during an assay. The reference receptor is further selected such that the rate constants for binding are substantially equivalent. The term "substantially equivalent," refers to statistical equivalence. Accordingly, signal development at the reference and test zones is directly proportional to the amounts of analyte receptor conjugate bound to the reference and test zones, respectively. The present invention, therefore, is of substantial utility because signal development at the reference zone provides a means for qualitative and quantitative determinations of target analytes bound to the test zone substantially independent of normal variations in assay conditions.

Also, a method is provided for incorporating internal references in analyte-receptor assays wherein a suitable ligand conjugate receptor, selected to bind with a ligand conjugate, may be used as a reference receptor.

Further, in accordance with the invention, processes for the performance of analyte-receptor assays for the determination of target analytes are provided.

Figure 1 is a top view of a solid phase useful in the invention, having a discrete test zone and a discrete

4

reference zone for the determination of target analyte.

Figure 2 is a top view of a solid phase useful in the invention, having a discrete test zone and two discrete reference zones for the quantitation of a target analyte.

Figure 3 demonstrates the binding of target analyle, $^{125}$I-labeled HCG, to immobilized analyte receptor, anti-HCG antibody.

Figure 4 demonstrates the binding of analyte receptor conjugate, anti-LH conjugate, to the test zone to which is bound target analyte, LH.

Figure 5 demonstrates the binding of analyte receptor conjugate, anti-LH antibody conjugate, to the reference zone to which is bound reference receptor, anti-alkaline phosphatase antibody.

As indicated above, the present invention provides a method for the incorporation of internal references in an analyte-receptor assay for use in the qualitative and/or quantitative determination of a target analyte in a sample. Analyte-receptor assays for which the present invention is particularly useful are sequential analyte-receptor assays which use an analyte receptor capable of binding a target analyte in an amount directly proportional to the amount of target analyte in the sample and a signal producing system.

In accordance with the present invention, an internal reference is provided by binding a suitable reference receptor in a discrete reference zone on the solid phase, separate from the discrete test zone to which analyte receptor is bound. The reference receptor is selected to bind with an analyte receptor conjugate capable also of binding with the target analyte. The binding of analyte receptor conjugate to the reference receptor at the reference zone, however, is independent of the binding of analyte receptor conjugate to the target analyte. The reference receptor is further selected such that the rates of binding of the analyte receptor conjugate to the reference receptor and to the target analyte are directly proportional to the amounts of the reference receptor and target analyte bound to the solid phase during an assay, (i.e., first-order rates of binding), and the first-order rate constants are substantially equivalent. Such conditions are accomplished by use of analyte receptor conjugate concentrations in sufficient excess over concentrations of reference receptor and target analyte bound via the analyte receptor to the solid phase such that the empirically determined rates of binding of the analyte receptor conjugate to the reference receptor at the reference zone and the target analyte at the test zone are first-order rates. Additionally, signal development at the reference zone and the test zone is directly proportional to the amount of analyte receptor conjugate bound to the reference and test zones, respectively, on the solid phase.

The internal reference provided by the present invention is bound to a solid phase in a discrete reference zone. Also bound to the solid phase is a analyte receptor in a discrete test zone. The solid phase preferably is incorporated into an apparatus suitable for performing analyte-receptor assays. Such an apparatus is described in the U.S. Patent No. 4,632,901 and comprises, for example, a porous solid phase member such as a membrane or a filter having bound thereto at least one discrete test zone and at least one discrete reference zone in accordance with the present invention. Such an apparatus further comprises a means, operatively associated with the porous solid phase member, for facilitating the flow of a fluid sample and assay reagents through the porous solid phase member. Alternatively, the solid phase member may comprise a porous matrix in which analyte receptor-bound microspheres are entrapped within at least one discrete test zone and reference receptor-bound microspheres are entrapped within at least one discrete reference zone, in a manner essentially as described in EPO Application EP-A-0200381.

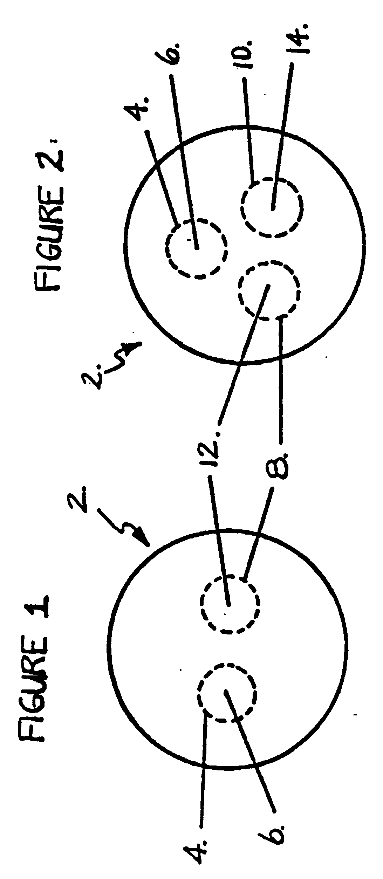

Turning now to Figure 1, there is shown a top view of a solid phase useful in the present invention for the determination of a target analyte in a sample. Thus, in Figure 1, a solid phase 2 is shown having a discrete test zone 4, to which is bound analyte receptor 6, and a discrete reference zone 8, to which is bound reference receptor 12. Figure 2 depicts a preferred solid phase system useful for the quantitation of target analyte. Thus, in Figure 2, a solid phase 2 is shown having a discrete test zone 4, to which is bound analyte receptor 6, and discrete reference zones 8 to 10 to which are bound reference receptors 12 and 14, respectively.

Sequential analyte-receptor assay processes may be performed in accordance with the present invention using an apparatus as described above. Such processes comprise introducing a liquid sample suspected of containing the target analyte to the solid phase thus binding the target analyte in the sample at the test zone. A solution containing an analyte receptor conjugate, i.e., a labeled receptor specific for the target analyte, is thereafter added to permit the detection and quantitation of the target analyte. The analyte receptor conjugate binds to the target analyte. The analyte receptor conjugate binds to the target analyte and to the reference receptor immobilized on the solid phase at rates which are directly proportional to the amounts of reference receptor and target analyte bound to the solid phase and having rate constants that are substantially equivalent. The addition of analyte receptor conjugate may be followed by brief incubation and washing steps to remove unbound analyte receptor conjugate from the solid phase. The presence of analyte receptor conjugate bound at the test and reference zones on the solid phase then is determined by signal development, permitting qualitative and quantitative determinations of the target analyte in the sample.

Alternatively, the sequential analyte-receptor assays provided by the invention may be performed by using a ligand conjugate receptor as a reference receptor. Accordingly, a solution containing analyte receptor conjugate, as well as ligand conjugate, is added to the solid phase following sample addition. The analyte receptor conjugate binds to target analyte at the test zone, and the ligand conjugate bind to ligand conjugate receptor at the reference zone, in amounts which are directly proportional to the amounts of target analyte and ligand conjugate receptor bound to the solid phase and having rate constants that are substantially equivalent.

In accordance with the present invention, suitable reference receptors are selected from target analyte, anti-enzyme antibody, anti-hapten antibody, anti-antibody antibody, anti-idiotype antibody, and ligand conjugate receptor.

Preferred for use as anti-enzyme antibody, anti-hapten antibody, anti-antibody antibody, anti-idiotype antibody and ligand conjugate receptor are monoclonal antibodies. However, those skilled in the art will recognize that polyclonal anti-sera may be utilized as reference receptors in the present invention. Also preferred for use as anti-antibody antibody and anti-idiotype antibody are antibodies which are specific for monoclonal antibodies.

Monoclonal antibodies and methods for their preparation are well known to those skilled in the art and need not be described; See, for example, Köhler and Milstein, Nature 256: 495-497 (1975).

The analyte receptors preferred for use in the invention are monoclonal antibodies. For example, in an immunometric assay of a sample for the presence of human chorionic gonadotropin (hCG), a monoclonal antibody specific for the alpha subunit of hCG is preferred for use as the analyte receptor bound to the solid phase and a labeled monoclonal antibody specific for the beta subunit of hCG is preferred for use as the analyte receptor conjugate. Additional target analytes may include IgE, luteinizing hormone (LH), follicle stimulating hormone (FSH) creatine kinase isoenzymes (CKMB and CKMM), alpha-feto protein (AFP) and hepatitis B surface antigen. For example, allergy specific IgE can be determined by using an allergen as an analyte receptor and a labeled anti-IgE antibody as an analyte receptor conjugate. However, those skilled in the art will readily appreciate that the present invention is useful for the determination of a variety of other target analytes.

The preferred labels used in the label component of the analyte receptor conjugates are enzyme labels. The use of enzymatic labels is well known to the art and generally is described in U.S. Patent No. 3,645,090. Detection of enzyme labels is accomplished by addition of a solution of a color-forming substrate to the solid phase to permit the substrate to react with the enzyme. Preferred are the enzyme labels alkaline phosphatase and horseradish peroxidase and the enzyme substrates indoxyl phosphate and tetramethyl benzidine. Those skilled in the art will appreciate that other enzymatic labels, as well as radionuclides, fluorescent agents, phosphorescent agents, and polymers containing dyes or chemiluminescent moieties also may be suitably utilized.

Analyte as a reference receptor provides a direct mechanism for calibration as it possesses the same binding characteristics as immobilized analyte at the test zone. Analyte may be immobilized on the reference zone either by direct immobilization or by immobilization of specific receptor-analyte complexes. Practical implementation requires consideration of cost, availability and immobilized stability of antigen or specific receptor-analyte complexes. In cases where such factors affect ease of manufacture or performance of the reference zone, the use of analyte as a reference receptor is less desirable.

Preferred for use as a reference receptor is anti-enzyme antibody. In accordance with the present invention, the rates of binding of analyte receptor conjugate to anti-enzyme reference receptor and to immobilized analyte are directly proportional to the amounts of immobilized anti-enzyme reference receptor and analyte on the solid phase and the rate constants must be substantially equivalent. The advantages of the use of an anti-enzyme receptor are cost and availability of reagent. Preferred anti-enzyme receptors are monoclonal antibodies which do not impair enzyme activity when bound to analyte receptor conjugates. Monoclonal antibodies may be further selected for stability properties such that the stability of the reference receptor and analyte receptor are comparable, provided that the requirement of substantially equivalent rate constants is met.

Also preferred as a reference receptor is anti-antibody antibody, having the same advantages as anti-enzyme antibody discussed above.

Also preferred for use as a reference receptor is anti-idiotype antibody. Anti-idiotype antibodies which bind to the variable region, but not to the hypervariable region of an antibody, possess the same advantages as anti-enzyme antibodies. However, additional advantages may be derived from the selection of anti-idiotype antibodies which bind to the hypervariable region of an antibody. Specifically, variations in the binding properties of the analyte receptor conjugate, attributable to instability of the receptor, may be reflected to the same degree in the binding of the conjugate to the reference receptor at the reference zone as in the binding of the analyte receptor conjugate to immobilized analyte at the test zone.

In certain applications, ligand conjugate receptor is preferred as a reference receptor. In circumstances in which ligand conjugate receptor is used as a reference receptor, a ligand conjugate will be used in addition to the analyte receptor conjugate. Ligands useful in the invention may be, for example, hen egg-white lysozyme, bovine serum albumin, horse radish peroxidase, bromelin, fluorescein and metal chelators. All the advantages relative to the use of anti-enzyme antibody as a reference receptor are applicable to the use of ligand conjugate receptor as a reference receptor. The rate for binding of ligand conjugate to ligand conjugate reference receptor must, of course, be directly proportional to the amount of immobilized ligand conjugate reference receptor and the rate for binding of analyte receptor conjugate to immobilized target analyte must be directly proportional to the amount of immobilized analyte on the solid phase. Additionally, the rate constants for binding of each species must be substantially equivalent. However, an advantage to be derived is that, independent of any differences between the second-order rate constants for binding of analyte receptor conjugate to the test zone and for binding of ligand conjugate to the reference zone, the first-order rate constant for binding of ligand conjugate to the reference zone may be independently varied such that it is substantially equivalent to the first-order rate constant for binding of analyte receptor conjugate to the test zone. This may be accomplished by appropriate adjustment of the ligand conjugate concentration subsequent to the manufacture of the test and reference zones.

Additionally, those skilled in the art will appreciate that the present invention suggests a method for providing internal references for use in analyte-receptor assays in which a target analyte in a sample is contacted with a solution comprising an analyte receptor conjugate and a ligand conjugate prior to introduction onto the solid phase. Accordingly, a ligand conjugate receptor is utilized as a reference receptor at the reference zone and an analyte receptor conjugate is contacted with a given volume of sample to permit the analyte receptor conjugate to pre-complex with the target analyte to form a target analyte complex. The target analyte complexes with the analyte receptor conjugate in an amount directly proportional to the amount of target analyte in the sample. Upon addition to the solid phase of the target analyte complex and the ligand conjugate, the target analyte complex binds to the analyte receptor at the test zone in an amount directly proportional to the amount of the target analyte complex added to the solid phase. The ligand conjugate binds to the ligand conjugate receptor at the reference zone in an amount directly proportional to the amount of ligand conjugate added to the solid phase. The constants of proportionality for the binding of the target analyte complex to the test zone and for the binding of ligand conjugate to the reference zone may be empirically determined because signal generation is in direct proportion to the amounts of target analyte complex and ligand conjugate bound to the test and reference zones, respectively. Thus, a quantitative relationship between signals measured at the reference and test zones can be established in accordance with the invention. The signal generated at the reference zone may be varied independently to equate with the signal generated at the test zone by a sample containing a particular analyte concentration. This may be accomplished by appropriate adjustment of the ligand conjugate concentration subsequent to the manufacture of the test and reference zones.

One will note the relationship between signal development at the test zone and the reference zone with respect to the foregoing assay process. In the case in which the first-order rate constants for the binding of the target analyte complex to the analyte receptor at the test zone and for the binding of the ligand conjugate to the ligand conjugate receptor at the reference zone are substantially equivalent, the relationship between signal development at the reference zone and the test zone will be substantially independent of normal variations in incubation time, temperature, and concentrations of reagents during the assay. Similarly, in the case where the binding of the target analyte complex to the analyte receptor at the test zone and the binding of the ligand conjugate to the ligand conjugate receptor at the reference zone are allowed to approach equilibrium, signal development will be substantially independent of variations in assay conditions.

A limitation of the present invention relative to the foregoing assay process in which analyte receptor conjugate pre-complexes with target analyte is that formation of the target analyte complex is a function of incubation time. The relationship between signals generated at the reference and test zones, therefore, is established for fixed times of incubation of sample and analyte receptor conjugate. However, by allowing complexation of the target analyte to analyte receptor conjugate to approach equilibrium the dependence on the incubation time becomes increasingly less important to the accuracy of the assay.

Additionally, those skilled in the art will appreciate that, consistent with the present disclosure, a plurality of internal references may be incorporated in single analyte-receptor assay systems for the qualitative and quantitative determination of a target analyte or multiple target analytes in a sample.

Qualitative and quantitative determinations of multiple target analytes having a common site to which a single analyte receptor conjugate is capable of binding, for example, LH and FSH, and CKMB and CKMM, may also be accomplished in accordance with the present invention. Further, it is possible, for example, to assay for IgE antibodies to multiple allergens using as the analyte receptor conjugate an antibody, preferably a monoclonal antibody, to the Fc portion of IgE. In such determinations, the reference zone or zones define the signal development for specific analyte concentrations relative to each target analyte. In the case of quantitative determinations, two internal references define the slope and intercept of the signal development as a function of target analyte concentration. Because the test zones may have different rates for binding their respective target analytes, the slope and intercept of signal development as a function of target analyte concentration may be different for each target analyte using the same two references. Therefore, the analyte-assigned values for the reference zones are specifically assigned for each of the analytes.

Alternatively, multiple target analytes can be determined using an equal multiplicity of analyte receptor conjugates and a ligand conjugate, in accordance with the present invention. In this application, the concentrations of each of the analyte receptor conjugate and of the ligand conjugate must be adjusted such that their respective rate constants for binding to each target analyte and to reference receptor are substantially equivalent. In cases where the assay process involves incubation prior to introduction on to the solid phase of sample containing multiple analytes and multiple analyte receptor conjugates, the signal generated by ligand conjugate bound to the reference zone will have a fixed relationship to specific sample concentrations of each analyte and the signals generated by such analyte bound to test zones.

Those skilled in the art will appreciate that the present invention is applicable to a variety of analyte-receptor assays. For example, the present invention is applicable to assays in which the target analyte is a specific antibody in a sample, such as assays for antibodies associated with immune responses to diseases and assays for allergy specific antibodies, particularly radio-allergo-sorbent test (RAST) assays. In the case of such assays, the analyte receptor bound to the test zone may be an antigen and the analyte receptor conjugate may be a labeled anti-antibody. Additionally, the present invention suggests the use of internal references, in accordance with this disclosure, for assays involving nucleic acid probe technology. In the case of assays involving nucleic acid probe technology, the analyte receptor bound to the test zone may be a nucleic acid sequence complementary to a portion of the nucleic acid sequence of the target analyte and the analyte receptor conjugate may be a labeled nucleic acid sequence complementary to a different portion of the nucleic acid sequence of the target analyte.

To further define the invention, the conditions necessary to establish the requisite correspondence between target analyte concentration and signal development within the assay range are discussed. As noted above, the target analyte must be bound to an analyte receptor in the test zone in an amount that is directly proportional to the initial amount of analyte in the sample. Additionally, the rate of binding of analyte receptor conjugate to the immobilized target analyte and the reference receptor must be in direct proportion to the amounts of these species immobilized at their respective test and reference zones and the rate constants must be substantially equivalent; the substantial equivalence of rate constants must be maintained under normal temperature variations that may occur during the performance of the assay. Accordingly, the amount of analyte receptor conjugate immobilized at the test zone is directly proportional to the original analyte concentration in the sample. Additionally, the amount of reference receptor bound at the reference zone is selected such that it binds the same amount of analyte receptor conjugate as is bound by a selected amount of target analyte bound at the test zone. This, in turn, represents a specific concentration of target analyte in a sample. Finally, signal generation must be in direct proportion to the amounts of analyte receptor conjugate bound to the reference and test zones.

Consequently, if the above conditions are satisfied, the target analyte concentration in a sample will be directly proportional to the analyte-specific signal generated as a result of the assay of the sample. Furthermore, the slope and intercept of the signal as a function of the target analyte concentration may be defined by internal references, as provided, which are representative of different analyte concentrations. The use of one internal reference represents the assay response for a particular concentration of analyte within the assay range and thus can be used as a qualitative calibration point; the use of two or more internal references may be used for quantitative calibrations. In either a qualitative or quantitative mode, the internal references maintain their correspondence to signals generated by fixed analyte concentrations in response to time, temperature and concentration variations during the binding of analyte receptor conjugate and during signal development.

In order to assign an analyte value to the reference zone it is necessary to first determine the signal level produced at the test zone, under a specified set of assay conditions, by a sample containing a given analyte concentration. Under the same set of conditions the amount of the reference receptor bound on the reference zone is adjusted to provide a substantially equivalent signal.

The following non-limiting examples are provided to further illustrate the invention.

Example 1

Determination of Binding of $^{125}$I-HCG To Immobilized Anti-HCG Receptor

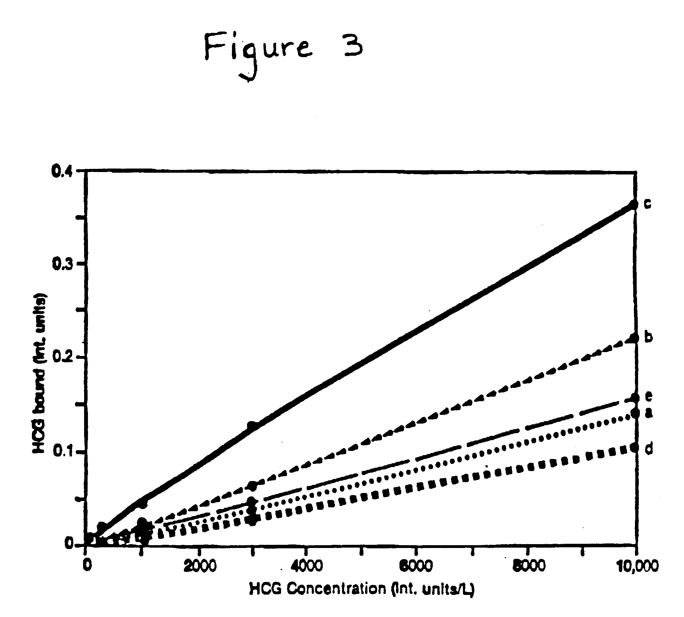

In several separate experiments fixed volumes of urine or serum containing a known range of HCG concentrations were added to the solid phase of assay devices, constructed in accordance with the invention, having a discrete test zone comprising anti-HCG receptor immobilized directly on the porous support material. The anti-HCG receptor was a monoclonal antibody specific for the alpha subunit of HCG. As shown by Figure 3 and Table I, the binding of $^{125}$IHCG to the immobilized anti-HCG receptor is directly proportional to the solution HCG concentration for a specified sample volume for concentrations of HCG up to 3000 int. units/L.

Results of Linear Regression of Bound $^{125}$I-Labeled
HCG vs Amount of HCG Added in Different Volumes
Of Urine and Serum

| Sample vol, mL | Slope, L | | y-intercept milli-int. units | | Syx milli-int. units |
|---|---|---|---|---|---|
| | Mean | SD | Mean | SD | |
| **Urine** | | | | | |
| 0.25* | 13.8 | 1.7 | 0.4 | 7.7 | 6.1 |
| 0.50* | 22.4 | 2.6 | -0.6 | 12.4 | 9.7 |
| 1.0* | 41.7[a] | 4.5[a] | 1.9[a] | 7.4[a] | 5.3[a] |
| **Serum** | | | | | |
| 0.25* | 10.7 | 1.0 | 0.1 | 5.0 | 3.9 |
| 0.50* | 16.0 | 2.2 | 0.05 | 10.6 | 8.3 |

n=50 each.

[a] Excluding data (n=10) from binding of HCG added at 10,000 int. units/L.

*In Figure 3, which graphically shows these results, a-c refers to urine samples of 0.25, 0.50 and 1.0 ml, respectively; d and e refers to serum samples of 0.25 and 0.50 ml, respectively.

Example 2

Determination of Rate Constants for Binding of Anti-LH Conjugate to Test and Reference Zones

The solid phase used in Example 3 was prepared as follows. Anti-LH antibody was adsorbed to latex microspheres and a suspension of such microspheres deposited upon a porous nylon membranes solid support to create a discrete test zone. Reference receptor in the form of anti-alkaline phosphatase antibody was diluted with bovine serum albumin, adsorbed to latex microspheres, and deposited upon a porous solid support to create a discrete reference zone. The dilutions of anti-alkaline phosphatase with bovine serum albumin were further selected such that the rate of binding of anti-LH conjugate to the reference zone was

10

directly proportional to the amount of anti-alkaline phosphatase in the reference zone and such that the rate constant for this binding was independent of such amount of anti-alkaline phosphatase.

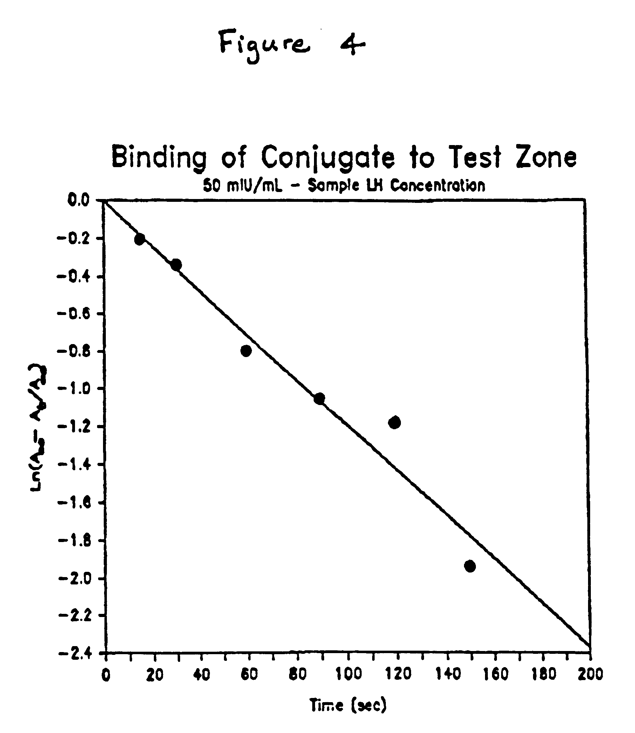

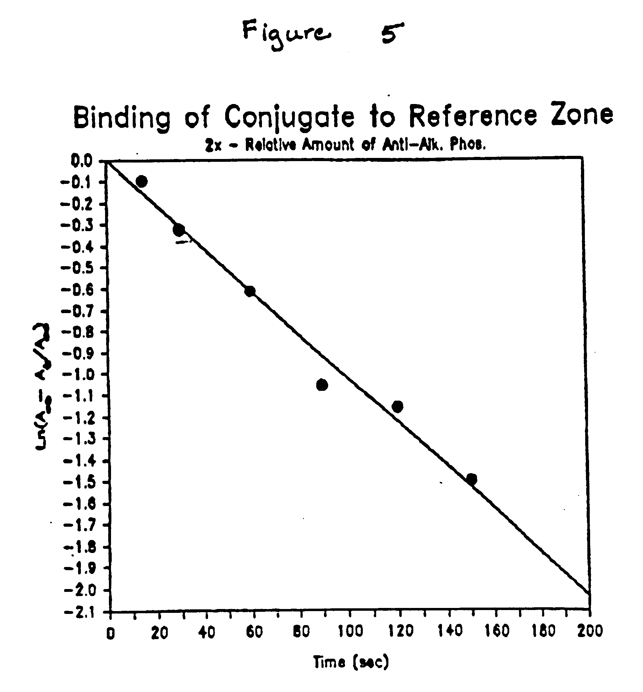

Using the solid phases prepared above, the rate constants for binding of anti-LH antibody, conjugated to alkaline phosphatase, to immobilized LH antigen-anti-LH antibody in the test zone and for binding to immobilized anti-alkaline phosphatase antibody in the reference zone were measured. The rate constants were measured as a function of anti-alkaline phosphatase antibody amount in the reference zone and as a function of the amount of LH immobilized by anti-LH antibody in the test zone. As shown in Table II, the rate constants for binding were found to be substantially equivalent for all cases of binding to both the test zone and reference zone. Further, as shown in Figures 4 and 5, the rate constants are pseudo-first-order constants. Because the rate constants are psuedo-first-order and substantially equivalent, they remain substantially equivalent independent of variation of incubation time and conjugate concentration.

## TABLE II

### Rate Constants for Binding of Anti-LH Conjugate to Reference Zone and Test Zone

| Relative Amount of Reference Receptor (Anti-Alkaline Phosphatase Antibody) | First Order Rate Constant (Mean ± 2SD) |
|---|---|
| 1x | $(7.5 \pm 2.0) \times 10^{-3} s^{-1}$ |
| 2x | $(10.2 \pm 1.4) \times 10^{-3} s^{-1}$ |
| 4x | $(7.4 \pm 1.4) \times 10^{-3} s^{-1}$ |

| LH Sample Concentration (mIU/ML | First Order Rate Constant (Mean ± 2SD) |
|---|---|
| 25 | $(7.3 \pm 2.4) \times 10^{-3} s^{-1}$ |
| 50 | $(11.8 \pm 2.6) \times 10^{-3} s^{-1}$ |
| 100 | $(10.3 \pm 5.0) \times 10^{-3} s^{-1}$ |

Example 3

Assay devices as previously described were prepared with one test zone and two reference zones on each solid phase. The reference zones were assigned analyte values by measuring their diffuse reflectance density with a reflectometer and then comparing their signal levels with those from assays of samples of known analyte concentration under equivalent assay conditions. Three unknown samples were then run with the analyte-value-assigned reference zones and the reflectance measured. The analyte values of the unknown samples were calculated by linear regression from the slop and intercept determined by the reflection densities from the two reference zones within a single device and compared to assay values determined using a commercially available quantitative assay (Tandem®-E HCG, Hybritech Incorporated, San Diego, CA 92126). As shown in Table III the quantitative values determined with the assay using internal references are comparable to the values determined by using the commercially available quantitative assay.

EP 0 253 464 B1

## Table III

### Quantitation of Serum Samples Containing Unknown HCG Antigen Concentrations

| HCG Conc.*<br>(mIU/mL) | TEST ZONE | | LOW REFERENCE ZONE<br>(29 mIU/mL) | HIGH REFERENCE ZONE<br>(215 mIU/mL) |
|---|---|---|---|---|
| | Reflection<br>Density<br>(K/S) | Assay<br>Value<br>(mIU/ml) | Reflection<br>Density<br>(K/S) | Reflection<br>Density<br>(K/S) |
| 51 | 0.361 | 46 | 0.243 | 1.502 |
| | 0.352 | 54 | 0.179 | 1.445 |
| | 0.388 | 60 | 0.184 | 1.416 |
| | 0.311 | 45 | 0.197 | 1.500 |
| | 0.392 | 60 | 0.197 | 1.384 |
| | 0.301 | 41 | 0.222 | 1.473 |
| | 0.397 | 57 | 0.207 | 1.466 |
| | Avg. ± S.D. | 52 ± 8 | | |
| 108 | 0.659 | 91 | 0.209 | 1.552 |
| | 0.891 | 129 | 0.186 | 1.496 |
| | 0.831 | 108 | 0.249 | 1.698 |
| | 0.846 | 121 | 0.220 | 1.490 |
| | 0.752 | 112 | 0.198 | 1.445 |
| | 0.617 | 80 | 0.209 | 1.692 |
| | 0.641 | 92 | 0.220 | 1.461 |
| | 0.817 | 119 | 0.220 | 1.457 |
| | 0.641 | 94 | 0.221 | 1.421 |
| | 0.752 | 109 | 0.223 | 1.451 |
| | Avg. ± S.D. | 106 ± 16 | | |
| 157 | 1.006 | 146 | 0.221 | 1.472 |
| | 1.106 | 160 | 0.230 | 1.471 |
| | 0.908 | 138 | 0.223 | 1.397 |
| | 1.287 | 190 | 0.220 | 1.453 |
| | 1.122 | 182 | 0.241 | 1.312 |
| | 1.145 | 177 | 0.229 | 1.382 |
| | 1.129 | 152 | 0.248 | 1.582 |
| | 1.046 | 153 | 0.226 | 1.458 |
| | 1.055 | 167 | 0.245 | 1.338 |
| | Avg. ± S.D. | 163 ± 17 | | |

*HCG determined by a commercially available assay for the quantitation of HCG. (Tandem-E® HCG Assay, Hybritech Incorporated, San Diego, CA.)

## Claims

**1.** A method for providing internal references for use in a sequential analyte-receptor assay for the determination of one or more target analytes in a sample, the assay employing a signal producing system and an analyte receptor capable of binding the target analyte in an amount directly proportional to the amount of the target analyte in the sample, which comprises:
binding to a solid phase, at a discrete reference zone separate from a discrete test zone to which the analyte receptor is bound, a reference receptor selected to bind with an analyte receptor conjugate which is capable of complexation with the target analyte;

12

EP 0 253 464 B1

and whereby signal development at the reference zone and the test zone is directly proportional to the amount of the analyte receptor conjugate bound to the reference and test zones, characterised in that the rates of binding of the analyte receptor conjugate to the reference receptor and to the target analyte are directly proportional to the amounts of the reference receptor and the target analyte bound to the solid phase, and the rate constants are substantially equivalent.

2. A method of claim 1 wherein a single target analyte is to be detected.

3. A method according to Claim 1 or claim 2 in which said reference receptor is a target analyte, an anti-enzyme antibody, an anti-hapten antibody, an anti-antibody antibody or an anti-idiotype antibody.

4. A method as claimed in Claim 3 in which the anti-enzyme antibody, said anti-hapten antibody, said anti-antibody antibody or said anti-idiotype antibody is a monoclonal antibody.

5. A method as claimed in Claim 3 in which the anti-antibody or the anti-idiotype antibody are specific for a monoclonal antibody.

6. A method according to Claim 1 or claim 2 in which said analyte receptor is a monoclonal antibody.

7. A method as claimed in Claim 1 or claim 2 in which the analyte receptor is a monoclonal antibody and the target analyte is HCG, LH, FSH, CKMB, CKMM, AFP or hepatitis B surface antigen.

8. A method as claimed in Claim 1 or claim 2 in which the analyte receptor is an allergen and the target analyte is allergen-specific IgE.

9. A method for providing internal references for use in a sequential analyte-receptor assay for the determination of one or more target analytes in a sample, said assay employing a signal producing system and an analyte receptor capable of binding the target analyte in an amount directly proportional to the amount of the target analyte in the sample, which comprises:
binding to a solid phase, at a discrete reference zone separate from a discrete test zone to which the analyte receptor is bound, a ligand conjugate reference receptor selected to bind with a ligand conjugate;
and whereby signal development at the reference zone is directly proportional to the amount of the ligand conjugate bound to the reference zone and signal development at said test zone is directly proportional to the amount of the analyte receptor conjugate bound to said test zone, characterised in that the rate of binding of the ligand conjugate to the reference receptor and the rate of binding of an analyte receptor conjugate to the target analyte are directly proportional to the amount of the reference receptor and the target analyte bound to the solid phase, and the rate constants are substantially equivalent.

10. A method of claim 9 wherein a single target analyte is to be detected.

11. A method as claimed in Claim 9 or claim 10 in which the ligand is a protein, enzyme, hapten or oligonucleotide.

12. A method as claimed in claim 11 in which the ligand is hen egg-white lysozyme, bovine serum albumin, horseradish peroxidase, bromelin, fluorescein or a metal chelator.

13. A method as claimed in claim 9 or claim 10 in which the reference receptor and said analyte receptor are monoclonal antibodies.

14. A method according to claim 1 or 9 in which the label component of the analyte receptor conjugate is an enzyme.

15. A method as claimed in Claim 14 in which the enzyme is alkaline phosphatase or horseradish peroxidase.

16. An analyte-receptor assay process using the method of claim 1 for the determination of a target analyte

13

in a sample which comprises:

a) introducing the sample suspected of containing the target analyte onto a solid phase comprising a discrete test zone to which an analyte receptor capable of binding with the target analyte is bound, the solid phase further comprising at least one discrete reference zone to which a reference receptor is bound, the analyte receptor being bound in a manner such that a direct proportion of the amount of the target analyte in the sample binds to the test zone;

b) adding a solution of analyte receptor conjugate, the analyte receptor conjugate being labeled to permit detection by signal development, in order to bind the analyte receptor conjugate to the target analyte and to the reference receptor such that the' rates of binding of the analyte receptor conjugate to the test zone and to said reference zone are directly proportional to the amounts of the target analyte and the reference receptor bound to the solid phase and the rate constants are substantially equivalent; and

c) determining signal development generated by the analyte receptor conjugate bound to the test and reference zones.

17. An analyte-receptor assay process using the method of claim 9 for the determination of a target analyte in a sample comprising:

a) introducing the sample suspected of containing the target analyte onto a solid phase comprising a discrete test zone to which an analyte receptor capable of binding the target analyte is bound, the solid phase further comprising at least one discrete reference zone to which a ligand conjugate reference receptor is bound, the analyte receptor being bound such that a direct proportion of the amount of said target analyte in said sample binds to said test zone;

b) adding a solution comprising analyte receptor conjugate and ligand conjugate, the analyte receptor conjugate and the ligand conjugate being labeled to permit detection by signal development, in order to bind said analyte receptor conjugate to said target analyte and said ligand conjugate to the reference receptor such that the rates of binding of the analyte receptor conjugate to the test zone and said ligand conjugate to the reference zone are directly proportional to the amounts of the target analyte and the reference receptor bound to the solid phase and the rate constants are substantially equivalent; and

c) determining signal development generated by the analyte receptor conjugate bound to the test zone and the ligand conjugate bound to the reference zone.

18. A method as claimed in Claim 1 or 9 in which at least two reference receptors are bound to said solid phase.

19. A process as claimed in Claim 16 or 17 in which the solid phase comprises at least two reference receptors.

20. A method as claimed in claim 1 for providing internal references for use in a sequential analyte-receptor assay for the determination of multiple target analytes in a sample wherein the target analytes have a common site to which an analyte receptor conjugate is capable of binding, and wherein the assay employs a signal producing system and analyte receptors capable of binding the target analytes in amounts directly proportional to the amounts of the target analytes in the sample, which comprises:

binding to a solid phase, at a discrete reference zone separate from a discrete test zone to which the analyte receptors are bound, at least one reference receptor selected to bind with the analyte receptor conjugate such that the rates of binding of the analyte receptor conjugate to the reference receptor and to the target analytes are directly proportional to the amounts of the reference receptor and the target analytes bound to the solid phase; the rate constants are substantially equivalent; and

whereby signal development at the reference zone and the test zones is directly proportional to the amounts of the analyte receptor conjugate bound to the reference and test zones.

21. A method as claimed in Claim 20 in which the target analytes are FSH and LH, or CKMB and CKMM.

22. A method as claimed in claim 9 for providing internal references for use in a sequential analyte-receptor assay for the determination of multiple target analytes in a sample wherein the assay employs a signal producing system, a ligand conjugate, analyte receptors capable of binding said target analytes in amounts directly proportional to the amounts of the target analytes in said sample, and analyte receptor conjugates in a number equal to the number of target analytes to be determined, which comprises:

binding to a solid phase, at a discrete reference zone separate from a discrete test zone to which the analyte receptors are bound, a ligand conjugate reference receptor selected to bind with the ligand conjugate, said ligand conjugate and said analyte receptor conjugates being further selected such that the rates of binding of the ligand conjugate to the reference receptor and the analyte receptor conjugates to the target analytes are directly proportional to the amounts of the reference receptor and the target analytes bound to the solid phase, the rate constants for binding of the ligand conjugate to said reference receptor and for binding of said analyte receptor conjugates to the target analytes are substantially equivalent;

and whereby signal development at the reference zone and the test zones are directly proportional to the amount of the ligand conjugate bound to the reference zone and to the amounts of the analyte receptor conjugates bound to the test zones.

23. A method according to Claim 22 in which said target analytes are FSH and LH or CKMB and CKMM.

24. A method for providing internal references for use in an analyte-receptor assay for the determination of one or more target analytes in a sample wherein said assay employs an analyte-receptor conjugate, said analyte receptor conjugate being pre-complexed with said target analyte to form a target analyte complex in an amount directly proportional to the amount of said target analyte in said sample, an analyte receptor capable of binding said target analyte complex in an amount directly proportional to the amount of said target analyte complex, a ligand conjugate and a signal producing system, said method comprising:

binding to a solid phase, at a discrete reference zone separate from a discrete test zone to which analyte receptor is bound, a ligand conjugate reference receptor selected to bind with a ligand conjugate;

and whereby signal development at the reference zone is directly proportional to the amount of the ligand conjugate bound to the reference zone and signal development at the test zone is directly proportional to the amount of the target analyte complex bound to the test zone, characterised in that said reference receptor and said analyte receptor are further selected such that the rate of binding for said ligand conjugate to said reference receptor and the rate of binding for said target analyte complex to said analyte receptor are directly proportional to the amounts of said ligand conjugate and said target analyte complex.

25. A method of claim 24 wherein a single target analyte is to be detected.

26. A method according to Claim 24 in which said ligand is selected from hen egg-white lysozyme, bovine serum albumin, horseradish peroxidase, bromelin, fluorescein and metal chelators.

27. A method according to Claim 24 wherein the label component of said analyte receptor conjugate and said ligand conjugate is selected from an enzyme, a radionuclide, a fluorescent agent, a phosphorescent agent, or a polymer containing dyes or a chemiluminescent moieties.

28. A method according to Claim 27 wherein said label is an enzyme.

29. A method according to Claim 28 wherein said enzyme is alkaline phosphatase or horseradish peroxidase.

30. A method as claimed in claim 24 for providing internal references for use in an analyte-receptor assay for the determination of multiple target analytes in a sample wherein said assay employs analyte-receptor conjugates in a number equal to the number of target analytes to be determined, the analyte receptor conjugates being pre-complexed with the target analytes to form target analyte complexes in amounts directly proportional to the amounts of the target analytes in the sample, analyte receptors capable of binding the target analyte complexes in amounts directly proportional to the amounts of said target analyte complexes, a ligand conjugate and a signal producing system, said method comprising:

binding to a solid phase, at a discrete reference zone separate from a discrete test zone to which the analyte receptors are bound, a ligand conjugate reference receptor selected to bind the ligand conjugate, said reference receptor and said analyte receptors being further selected such that the rate of binding the ligand conjugate to the reference receptor and the rates of binding the target analyte complexes to the analyte receptors are directly proportional to the amounts of the ligand conjugate and the target analyte complexes; and

whereby signal development at the reference zone is directly proportional to the amount of the ligand conjugate bound to said reference zone and signal development at the test zones is directly proportional to the amounts of the target analyte complexes bound to the test zones.

31. An apparatus for use in an analyte-receptor assay for the determination of a target analyte in a sample comprising:

a porous solid phase member comprising a discrete test zone to which is bound analyte receptor capable of binding said target analyte, and at least one discrete reference zone to which is bound reference receptor, said reference receptor being selected to bind with an analyte receptor conjugate capable of binding with said target analyte, and whereby signal development at said reference zone and said test zone is directly proportional to the amount of said analyte receptor conjugate bound to said reference zone and said test zone;

characterised in that said reference receptor is further selected such that the rates for binding of said analyte receptor conjugate to said reference receptor and to said target analyte are directly proportional to the amounts of said reference receptor and said target analyte bound to said solid phase, and such that the rate constants are substantially equivalent.

32. An apparatus according to Claim 31 wherein said solid phase member comprises a porous matrix in which analyte receptor-bound microspheres are entrapped within said discrete test zone and reference receptor-bound microspheres are entrapped within said discrete reference zone.

33. An apparatus as claimed in Claim 31 or 32 comprising a means, operatively associated with said porous solid phase member, for facilitating the flow of said fluid sample and assay reagents through said porous solid phase member.

**Revendications**

1. Méthode pour procurer des références internes qui peuvent être utilisées dans un dosage substance à analyser-récepteur séquentiel pour la détermination d'une ou de plusieurs substances-cibles à analyser dans un échantillon, le dosage utilise un système producteur d'un signal et un récepteur pour la substance à analyser capable de lier la substance-cible en quantité directement proportionnelle à la quantité de substance-cible à analyser dans l'échantillon, qui comprend :

la fixation à une phase solide, en une zone de référence discrète, distincte d'une zone d'essai discrète à laquelle est lié le récepteur pour la substance à analyser, d'un récepteur de référence sélectionné pour lier un conjugué du récepteur pour la substance à analyser qui est capable de former un complexe avec la substance-cible;

et où le développement de signal à la zone de référence et la zone d'essai est directement proportionnel à la quantité de conjugué du récepteur pour la substance à analyser liée aux zones d'essai et de référence, caractérisée en ce que les vitesses de liaison du conjugué du récepteur pour la substance à analyser au récepteur de référence et à la substance-cible à analyser sont directement proportionnelles aux quantités du récepteur de référence et de la substance-cible à analyser liées à la phase solide, et en ce que les constantes de vitesse sont substantiellement équivalentes.

2. Méthode selon la revendication 1, dans laquelle une seule substance-cible à analyser doit être détectée.

3. Méthode selon la revendication 1 ou 2, dans laquelle ledit récepteur de référence est une substance-cible à analyser, un anticorps anti-enzyme, un anticorps anti-haptène, un anticorps anti-anticorps ou un anticorps anti-idiotype.

4. Méthode selon la revendication 3, dans laquelle ledit anticorps anti-enzyme, ledit anticorps anti-haptène, ledit anticorps anti-anticorps ou ledit anticorps anti-idiotype est un anticorps monoclonal.

5. Méthode selon la revendication 3, dans laquelle l'anticorps anti-idiotype ou anti-anticorps est spécifique pour un anticorps monoclonal.

6. Méthode selon la revendication 1 ou 2, dans laquelle ledit récepteur pour la substance à analyser est un anticorps monoclonal.

7. Méthode selon la revendication 1 ou 2, dans laquelle le récepteur pour la substance à analyser est un anticorps monoclonal et la substance-cible à analyser est HCG, LH, FSH, CKMB, CKMM, AFP ou l'antigène de surface de l'hépatite B.

8. Méthode selon la revendication 1 ou 2, dans laquelle le récepteur pour la substance à analyser est un allergène et la substance-cible à analyser est une IgE spécifique d'allergènes.

9. Méthode pour procurer des références internes qui peuvent être utilisées dans un dosage substance à analyser-récepteur séquentiel pour la détermination d'une ou de plusieurs substances-cibles à analyser dans un échantillon, ledit dosage utilise un système producteur d'un signal et un récepteur pour la substance à analyser capable de lier la substance-cible à analyser en quantité directement proportionnelle à la quantité de la substance-cible à analyser dans l'échantillon, qui comprend :

la liaison à une phase solide, en une zone de référence discrète, distincte d'une zone d'essai discrète à laquelle est lié le récepteur pour la substance à analyser, d'un récepteur de référence pour le conjugué du ligand sélectionné pour lier un conjugué du ligand;

et où le développement du signal à la zone de référence est directement proportionnel à la quantité du conjugué du ligand liée à la zone de référence et le développement du signal à ladite zone d'essai est directement proportionnel à la quantité de conjugué du récepteur pour la substance à analyser liée à ladite zone d'essai, caractérisée en ce que la vitesse de liaison du conjugué du ligand au récepteur de référence et la vitesse de liaison du conjugué du récepteur pour la substance à analyser à la substance-cible à analyser sont directement proportionnelles à la quantité du récepteur de référence et de la substance-cible à analyser liées à la phase solide, et les constantes de vitesse sont substantiellement équivalentes.

10. Méthode selon la revendication 9, dans laquelle une seule substance-cible à analyser doit être détectée.

11. Méthode selon la revendication 9 ou 10, dans laquelle le ligand est une protéine, une enzyme, un haptène ou un oligonucléotide.

12. Méthode selon la revendication 11, dans laquelle le ligand est le lysozyme du blanc d'oeuf de poule, l'albumine de sérum bovin, la peroxydase de raifort, la broméline, la fluoroscéine ou un chélateur de métal.

13. Méthode selon la revendication 9 ou 10, dans laquelle le récepteur de référence et ledit récepteur pour la substance à analyser sont des anticorps monoclonaux.

14. Méthode selon la revendication 1 ou 9, dans laquelle le composant marqueur du conjugué du récepteur pour la substance à analyser est une enzyme.

15. Méthode selon la revendication 14, dans laquelle l'enzyme est la phosphatase alcaline ou la peroxydase de raifort.

16. Procédé de dosage substance à analyser-récepteur utilisant la méthode selon la revendication 1 pour la détermination d'une substance-cible à analyser dans un échantillon, qui comprend :

a) introduction de l'échantillon susceptible de contenir la substance-cible à analyser sur une phase solide qui comprend une zone d'essai discrète à laquelle est lié un récepteur pour la substance à analyser capable de lier la substance-cible à analyser, la phase solide comprenant en plus au moins une zone de référence discrète à laquelle est lié un récepteur de référence, le récepteur pour la substance à analyser étant lié de façon à ce qu'une proportion directe de la quantité de substance-cible à analyser dans l'échantillon se fixe à la zone d'essai;

b) addition d'une solution de conjugué du récepteur pour la substance à analyser, le conjugué du récepteur pour la substance à analyser étant marqué pour permettre une détection par un développement d'un signal afin de lier le conjugué du récepteur pour la substance à analyser à la substance-cible et au récepteur de référence de façon à ce que les vitesses de liaison du conjugué du récepteur pour la substance à analyser à la zone d'essai et à ladite zone de référence soient directement proportionnelles aux quantités de la substance-cible à analyser et du récepteur de référence liées à la phase solide et les constantes de vitesse sont substantiellement équivalentes; et

c) détermination du développement d'un signal produit par le conjugué du récepteur pour la substance à analyser lié aux zones de référence et d'essai.

17. Procédé de dosage substance à analyser-récepteur qui utilise une méthode selon la revendication 9 pour la détermination d'une substance-cible dans un échantillon, qui comprend :

a) introduction d'un échantillon susceptible de contenir la substance-cible à analyser sur une phase solide qui comprend une zone d'essai discrète à laquelle est lié un récepteur pour la substance à analyser capable de lier la substance-cible à analyser, la phase solide comprenant en plus au moins une zone de référence discrète à laquelle est lié un récepteur de référence pour le conjugué du ligand, le récepteur pour la substance à analyser étant lié de façon à ce qu'une proportion directe de la quantité de ladite substance-cible dans ledit échantillon se fixe à ladite zone d'essai;

b) addition d'une solution comprenant du conjugué de récepteur pour la substance à analyser et du conjugué du ligand, le conjugué du récepteur pour la substance à analyser et le conjugué du ligand étant marqués pour permettre la détection par le développement d'un signal, afin de lier ledit conjugué du récepteur pour la substance à analyser à ladite substance-cible et ledit conjugué du ligand au récepteur de référence de façon à ce que les vitesses de liaison du conjugué du récepteur de la substance à analyser à la zone d'essai et dudit conjugué du ligand à la zone de référence sont directement proportionnelles aux quantités de la substance-cible et du récepteur de référence liées à la phase solide et les constantes de vitesse sont substantiellement équivalentes; et

c) détermination du développement d'un signal produit par le conjugué du récepteur de la substance à analyser lié à la zone d'essai et le conjugué du ligand lié à la zone de référence.

18. Méthode selon la revendication 1 ou 9, dans laquelle au moins deux récepteurs de référence sont liés à ladite phase solide.

19. Procédé selon la revendication 16 ou 17, dans lequel la phase solide comprend au moins deux récepteurs de référence.

20. Méthode selon la revendication 1 pour procurer des références internes qui peuvent être utilisées dans un dosage substance à analyser-récepteur séquentiel pour la détermination de substances-cibles multiples dans un échantillon où les substances-cibles ont un site commun auquel un conjugué du récepteur pour les substances à analyser est capable de se lier, et où le dosage emploie un système producteur d'un signal et des récepteurs pour les substances à analyser capable de lier les substances-cibles en quantités directement proportionnelles aux quantités des substances-cibles dans l'échantillon, qui comprend :

la liaison à une phase solide, à une zone de référence discrète, distincte d'une zone d'essai discrète à laquelle sont liés les récepteurs pour la substance à analyser, d'au moins un récepteur de référence sélectionné pour lier un conjugué de récepteur pour la substance à analyser de façon à ce que les vitesses de liaison du conjugué du récepteur pour les substances à analyser au récepteur de référence et aux substances-cibles soient directement proportionnelles aux quantités du récepteur de référence et des substances-cibles liées à la phase solide; les constantes de vitesse sont substantiellement équivalentes; et

où le développement du signal à la zone de référence et aux zones d'essai est directement proportionnel aux quantités du conjugué du récepteur pour la substance à analyser liées aux zones de référence et d'essai.

21. Méthode selon la revendication 20, dans laquelle les substances-cibles sont FSH et LH, ou CKMB et CKMM.

22. Méthode selon la revendication 9 pour procurer des références internes qui peuvent être utilisées dans un dosage substance à analyser-récepteur séquentiel pour la détermination de substances-cibles multiples dans un échantillon où le dosage fait appel à un système producteur d'un signal, un conjugué de ligand, des récepteurs pour les substances à analyser capables de lier lesdites substancescibles en quantités directement proportionnelles aux quantités des substances-cibles dans ledit échantillon, et des conjugués du récepteur pour la substance à analyser en nombre égal au nombre de substances-

18

cibles à déterminer, qui comprend :

la liaison à une phase solide, à une zone de référence discrète, distincte d'une zone d'essai discrète à laquelle sont liés les récepteurs pour la substance à analyser, d'un récepteur de référence pour conjugué du ligand sélectionné pour lier le conjugué du ligand, ledit conjugué du ligand et lesdits conjugués des récepteurs pour la substance à analyser étant sélectionnés en plus de façon à ce que les vitesses de liaison du conjugué du ligand au récepteur de référence et des conjugués des récepteurs pour les substances à analyser aux substances-cibles soient directement proportionnelles aux quantités du récepteur de référence et des substances-cibles liées à la phase solide, les constantes de vitesse pour la liaison du conjugué du ligand audit récepteur de référence et pour la liaison desdits conjugués des récepteurs pour les substances à analyser aux substances-cibles sont substantiellement équivalentes;

et où le développement du signal à la zone de référence et aux zones d'essai est directement proportionnel à la quantité du conjugué du ligand liée à la zone de référence et aux quantités des conjugués des récepteurs pour les substances à analyser liées aux zones d'essai.

23. Méthode selon la revendication 22, dans laquelle lesdites substances-cibles sont FSH et LH ou CKMB et CKMM.

24. Méthode pour procurer des références internes qui peuvent être utilisées dans un dosage substance à analyser-récepteur pour la détermination d'une ou de plusieurs substances-cibles dans un échantillon où ledit dosage utilise un conjugué du récepteur pour la substance à analyser, ledit conjugué du récepteur pour la substance à analyser pré-complexant ladite substance-cible pour former un complexe de substance-cible à analyser en quantité directement proportionnelle à la quantité de ladite substance-cible dans ledit échantillon, un récepteur pour la substance à analyser capable de lier ledit complexe de la substance-cible en quantité directement proportionnelle à la quantité dudit complexe de substance-cible, un conjugué du ligand et un système producteur d'un signal, ladite méthode comprenant :

la liaison à une phase solide, à une zone de référence discrète, distincte d'une zone d'essai discrète à laquelle est lié un récepteur pour la substance à analyser, d'un récepteur de référence pour conjugué du ligand sélectionné pour lier un conjugué du ligand;

et où le développement du signal à la zone de référence est directement proportionnel à la quantité du conjugué du ligand liée à la zone de référence et le développement du signal à la zone d'essai est directement proportionnel à la quantité du complexe de la substance-cible liée à la zone d'essai, caractérisée en ce que ledit récepteur de référence et ledit récepteur pour la substance à analyser sont sélectionnés en plus de façon à ce que la vitesse de liaison dudit conjugué du ligand audit récepteur de référence et la vitesse de liaison dudit complexe de la substance-cible audit récepteur pour la substance à analyser soient directement proportionnelles aux quantités dudit conjugué du ligand et dudit complexe de la substance-cible.

25. Méthode selon la revendication 24, dans laquelle on doit détecter une seule substance-cible.

26. Méthode selon la revendication 24, dans laquelle ledit ligand est choisi parmi le lysozyme de blanc d'oeuf de poule, l'albumine de sérum bovin, la peroxydase de raifort, la broméline, la fluorescéine et des chélateurs de métal.

27. Méthode selon la revendication 24, dans laquelle le composant marqué dudit conjugué du récepteur pour la substance à analyser et dudit conjugué du ligand est choisi parmi une enzyme, un radionucléide, un agent fluorescent, un agent phosphorescent, ou un polymère contenant des colorants ou des parties chimiluminescentes.

28. Méthode selon la revendication 27, dans laquelle ledit marqueur est une enzyme.

29. Méthode selon la revendication 28, dans laquelle ladite enzyme est une phosphatase alcaline ou une peroxydase de raifort.

30. Méthode selon la revendication 24 pour procurer des références internes qui peuvent être utilisées dans un dosage substance à analyser-récepteur pour la détermination de substances-cibles multiples dans un échantillon où ledit dosage utilise des conjugués de récepteurs pour la substance à analyser

EP 0 253 464 B1

en nombre égal au nombre de substances-cibles à déterminer, les conjugués de récepteurs pour la substance à analyser pré-complexant les substancescibles pour former des complexes de substances-cibles à analyser en quantités directement proportionnelles aux quantités de substances-cibles dans l'échantillon, des récepteurs pour substances à analyser capables de lier les complexes de substances-cibles en quantités directement proportionnelles aux quantités desdits complexes de substance-cible, un conjugué de ligand et un système producteur d'un signal, ladite méthode comprenant :

la liaison à une phase solide, en une zone de référence discrète, distincte d'une zone d'essai à laquelle sont liés des récepteurs pour les substances à analyser, d'un récepteur de référence pour conjugué du ligand sélectionné pour lier le conjugué du ligand, ledit récepteur de référence et lesdits récepteurs pour les substances à analyser étant sélectionnés en plus de façon à ce que la vitesse de liaison du conjugué du ligand au récepteur- de référence et les vitesses de liaison des complexes de substance-cible aux récepteurs pour des substances à analyser sont directement proportionnelles aux quantités du conjugué du ligand et aux complexes de substance-cible; et

où le développement du signal à la zone de référence est directement proportionnel à la quantité du conjugué du ligand liée à ladite zone de référence, et le développement du signal aux zones d'essai est directement proportionnel aux quantités des complexes de substance-cible liées aux zones d'essai.

31. Appareil qui peut être utilisé dans un dosage substance à analyser-récepteur pour la détermination d'une substance-cible dans un échantillon, comprenant :

un membre poreux de phase solide comprenant une zone d'essai discrète à laquelle est lié un récepteur pour la substance à analyser capable de lier ladite substance-cible, et au moins une zone de référence discrète à laquelle est lié un récepteur de référence, ledit récepteur de référence étant sélectionné pour lier un conjugué du récepteur pour la substance à analyser capable de lier ladite substance-cible, et où le développement de signal & ladite zone de référence et ladite zone d'essai est directement proportionnel a la quantité dudit conjugué du récepteur pour la substance à analyser liée à ladite zone de référence et ladite zone d'essai, caractérisé en ce que ledit récepteur de référence est sélectionné en plus de façon à ce que les vitesses de liaison dudit conjugué du récepteur pour la substance à analyser audit récepteur de référence et à ladite substance-cible à analyser sont directement proportionnelles aux quantités dudit récepteur de référence et de ladite substance-cible liées à ladite phase solide, et de façon à ce que les constantes de vitesse soient substantiellement équivalentes.

32. Appareil selon la revendication 31, dans laquelle ledit membre de phase solide comprend une matrice poreuse dans laquelle des microsphères fixant le récepteur pour la substance à analyser sont piégées dans ladite zone d'essai discrète et des microsphères fixant le récepteur de référence sont piégées dans ladite zone de référence discrète.

33. Appareil selon la revendication 31 ou 32, comprenant un moyen, associé opérativement avec ledit membre de phase solide poreuse, qui facilite le flux dudit échantillon liquide et des réactifs de dosages à travers ledit membre de phase solide poreuse.

**Patentansprüche**

1. Verfahren, um einen inneren Standard bereitzustellen zur Verwendung in einem mehrstufigen Analyt-Rezeptor-Assay für die Bestimmung eines oder mehrerer zu bestimmender Analyten in einer Probe, wobei in dem Assay ein signalerzeugendes System und ein Analyt-Rezeptor verwendet wird, der den zu bestimmenden Analyten in einer Menge binden kann, die direkt proportional ist zu der Menge des zu bestimmenden Analyten in der Probe, umfassend:

daß man an eine feste Phase in einem diskreten Referenzbereich getrennt von einem diskreten Testbereich, an den ein Analyt-Rezeptor gebunden ist, einen Referenz-Rezeptor bindet, der so ausgewählt ist, daß er mit einem Analyt-Rezeptor-Konjugat, das den zu bestimmenden Analyt komplexieren kann, bindet,

und wobei die Entwicklung des Signals im Referenzbereich und im Testbereich direkt proportional ist zu der Menge des Analyt-Rezeptor-Konjugats, das im Referenzbereich und im Testbereich gebunden wird, dadurch gekennzeichnet, daß die Bindungsraten des Analyt-Rezeptor-Konjugats an den Referenz-Rezeptor und an den zu bestimmenden Analyten direkt proportional sind zu den Mengen des Referenz-Rezeptors und des zu bestimmenden Analyten, der an die Festphase gebunden ist, und daß die Geschwindigkeitskonstanten im wesentlichen gleich sind.

20

**2.** Verfahren nach Anspruch 1, worin ein einzelner zu bestimmender Analyt nachgewiesen wird.

**3.** Verfahren nach Anspruch 1 oder Anspruch 2, worin der Referenz-Rezeptor ein zu bestimmender Analyt, ein Anti-Enzym-Antikörper, ein Anti-Hapten-Antikörper, ein Anti-Antikörper-Antikörper oder ein Anti-Idiotyp-Antikörper ist.

**4.** Verfahren nach Anspruch 3, worin der Anti-Enzym-Antikörper, der Anti-Hapten-Antikörper, der Anti-Antikörper-Antikörper oder der Anti-Idiotyp-Antikörper ein monoklonaler Antikörper ist.

**5.** Verfahren nach Anspruch 3, worin der Anti-Antikörper oder der Anti-Idiotyp-Antikörper spezifisch für einen monoklonalen Antikörper sind.

**6.** Verfahren nach Anspruch 1 oder Anspruch 2, worin der Analyt-Rezeptor ein monoklonaler Antikörper ist.

**7.** Verfahren nach Anspruch 1 oder Anspruch 2, worin der Analyt-Rezeptor ein monoklonaler Antikörper und der zu bestimmende Analyt HCG, LH, FSH, CKMB, CKMM, AFP oder Hepatitis-B-Oberflächen-Antigen ist.

**8.** Verfahren nach Anspruch 1 oder Anspruch 2, worin der Analyt-Rezeptor ein Allergen und der zu bestimmende Analyt ein allergiespezifischer IgE ist.

**9.** Verfahren, um einen inneren Standard zur Verwendung in einem mehrstufigen Analyt-Rezeptor-Assay für die Bestimmung eines oder mehrerer zu bestimmender Analyten in einer Probe zur Verfügung zu stellen, wobei bei dem Assay ein signalerzeugendes System und ein Analyt-Rezeptor, der den zu bestimmenden Analyten in einer solchen Menge binden kann, die direkt proportional ist zu der Menge des zu bestimmenden Analyten in der Probe, angewandt wird, umfassend:
daß man an eine Festphase in einem diskreten Referenzbereich getrennt von einem diskreten Testbereich, in dem der Analyt-Rezeptor gebunden ist, einen Ligand-Konjugat-Referenz-Rezeptor bindet, der ausgewählt ist, daß er mit einem Ligand-Konjugat bindet,
und wobei die Signalentwicklung im Referenzbereich direkt proportional ist zu der Menge des Ligand-Konjugats, das im Referenzbereich gebunden wird, und die Signalentwicklung im Testbereich direkt proportional ist zu der Menge des Analyt-Rezeptor-Konjugats, das im Testbereich gebunden wird, dadurch gekennzeichnet, daß die Bindungsrate des Ligand-Konjugats an den Referenz-Rezeptor und die Bindungsrate eines Analyt-Rezeptor-Konjugats an den zu bestimmenden Analyt direkt proportional zu der Menge des Referenz-Rezeptors und des zu bestimmenden Analyten, die an die Festphase gebunden sind, ist, und die Geschwindigkeitskonstanten im wesentlichen gleich sind.

**10.** Verfahren nach Anspruch 9, worin ein einzelner zu bestimmender Analyt nachgewiesen wird.

**11.** Verfahren nach Anspruch 9 oder Anspruch 10, worin der Ligand ein Protein, Enzym, Hapten oder Oligonukleotid ist.

**12.** Verfahren nach Anspruch 11, worin der Ligand Hühnereiweißlysozym, Rinderserumalbumin, Meerrettichperoxidase, Bromelin, Fluorescein oder ein Metallkomplexbildner ist.

**13.** Verfahren nach Anspruch 9 oder Anspruch 10, worin der Referenz-Rezeptor und der Analyt-Rezeptor monoklonale Antikörper sind.

**14.** Verfahren nach Anspruch 1 oder 9, worin die Markierungskomponente des Analyt-Rezeptor-Konjugats ein Enzym ist.

**15.** Verfahren nach Anspruch 14, worin das Enzym alkalische Phosphatase oder Meerrettichperoxidase ist.

**16.** Analyt-Rezeptor-Assay-Verfahren unter Verwendung des Verfahrens von Anspruch 1 zur Bestimmung eines zu bestimmenden Analyten in einer Probe, umfassend:
a) daß man die Probe, von der angenommen wird, daß sie den zu bestimmenden Analyten enthält, auf eine feste Phase aufbringt, die einen diskreten Testbereich, an den ein AnalytRezeptor, der mit dem zu bestimmenden Analyten binden kann, gebunden ist, umfaßt, wobei die feste Phase weiterhin mindestens einen diskreten Referenzbereich umfaßt, an den ein Referenz-Rezeptor gebunden ist, wobei der Analyt-Rezeptor in einer solchen Weise gebunden ist, daß die Menge an zu bestimmendem Analyten in der Probe im Testbereich direkt proportional bindet;

b) eine Lösung des Analyt-Rezeptor-Konjugats zugibt, wobei das Analyt-Rezeptor-Konjugat markiert ist, damit eine Signalentwicklung detektiert werden kann, um das Analyt-Rezeptor-Konjugat an den zu bestimmenden Analyten und den Referenz-Rezeptor zu binden, so daß die Bindungsraten des Analyt-Rezeptor-Konjugats im Testbereich und im Referenzbereich direkt proportional zu den Mengen an zu bestimmendem Analyt und Referenz-Rezeptor, die an die feste Phase gebunden sind, sind, und die Geschwindigkeitskonstanten im wesentlichen gleich sind; und

c) die Signalentwicklung, die durch das Analyt-Rezeptor-Konjugat, das im Test- und Referenzbereich gebunden ist, erzeugt wird, bestimmt.

17. Analyt-Rezeptor-Assay-Verfahren unter Verwendung des Verfahrens von Anspruch 9 für die Bestimmung eines zu bestimmenden Analyten in einer Probe, umfassend:

a) daß man die Probe, von der man annimmt, daß sie den zu bestimmenden Analyten enthält, auf eine Festphase aufbringt, die einen diskreten Testbereich, an den ein Analyt-Rezeptor gebunden ist, der den zu bestimmenden Analyten binden kann, umfaßt, wobei die feste Phase weiterhin mindestens einen diskreten Referenzbereich umfaßt, an den ein Ligand-Konjugat-Referenz-Rezeptor gebunden ist, wobei der Analyt-Rezeptor so gebunden wird, daß die Bindung direkt proportional ist zu der Menge an zu bestimmendem Analyt in der Probe, die im Testbereich gebunden wird;

b) eine Lösung, die ein Analyt-Rezeptor-Konjugat und ein Ligand-Konjugat umfaßt, zugibt, wobei das Analyt-Rezeptor-Konjugat und das Ligand-Konjugat markiert sind, damit die Signalentwicklung nachgewiesen werden kann, damit das Analyt-Rezeptor-Konjugat mit dem zu bestimmenden Analyten und das Ligand-Konjugat mit dem Referenz-Rezeptor so binden, daß die Bindungsraten des Analyt-Rezeptor-Konjugats im Testbereich und des Ligand-Konjugats im Referenzbereich direkt proportional sind zu den Mengen von zu bestimmendem Analyt und Referenz-Rezeptor, die an die feste Phase gebunden sind, und die Geschwindigkeitskonstanten im wesentlichen gleich sind; und

c) die durch im Testbereich gebundenes Analyt-Rezeptor-Konjugat und im Referenzbereich gebundenes Ligand-Konjugat erzeugte Signalentwicklung bestimmt.

18. Verfahren nach Anspruch 1 oder 9, worin mindestens zwei Referenz-Rezeptoren an die Festphase gebunden werden.

19. Verfahren nach Anspruch 16 oder 17, worin die feste Phase mindestens zwei Referenz-Rezeptoren umfaßt.

20. Verfahren nach Anspruch 1, um innere Standards zur Verwendung in einem mehrstufigen Analyt-Rezeptor-Assay fur die Bestimmung einer Vielzahl von zu bestimmenden Analyten in einer Probe bereitzustellen, wobei die zu bestimmenden Analyten eine gemeinsame Bindungsstelle haben, an die ein Analyt-Rezeptor-Konjugat binden kann, und wobei in dem Assay ein signalerzeugendes System und Analyt-Rezeptoren, die die zu bestimmenden Analyten in solchen Mengen binden können, die direkt proportional sind zu den Mengen an zu bestimmenden Analyten in der Probe, angewendet werden, umfassend:

daß man an eine Festphase in einem diskreten Referenzbereich getrennt von einem diskreten Testbereich, in dem die Analyt-Rezeptoren gebunden sind, mindestens einen Referenz-Rezeptor bindet, der so ausgewählt ist, daß er mit dem Analyt-Rezeptor-Konjugat so bindet, daß die Bindungsraten des Analyt-Rezeptor-Konjugats mit dem Referenz-Rezeptor und mit den zu bestimmenden Analyten direkt proportional sind zu den Mengen an Referenz-Rezeptor und zu bestimmenden Analyten, die an die Festphase gebunden sind; wobei die Geschwindigkeitskonstanten im wesentlichen gleich sind; und wobei die Signalentwicklung im Referenzbereich und in den Testbereichen direkt proportional ist zu den Mengen an Analyt-Rezeptor-Konjugat, das im Referenz- und Testbereich gebunden wird.

21. Verfahren nach Anspruch 20, worin die zu bestimmenden Analyten FSH und LH oder CKMB und CKMM sind.

22. Verfahren nach Anspruch 9, um innere Standards zur Verwendung in einem mehrstufigen Analyt-Rezeptor-Assay für die Bestimmung einer Vielzahl von zu bestimmenden Analyten in einer Probe

EP 0 253 464 B1

bereitzustellen, wobei in dem Assay ein signalerzeugendes System, ein Ligand-Konjugat, Analyt-Rezeptoren, die mit den zu bestimmenden Analyten in solchen Mengen binden können, die direkt proportional zu den Mengen der zu bestimmenden Analyten in der Probe sind, und Analyt-Rezeptor-Konjugate in einer Anzahl, die gleich ist der Zahl von zu bestimmenden Analyten, angewendet werden, umfassend:

daß man an eine Festphase in einem diskreten Referenzbereich getrennt von einem diskreten Testbereich, in dem die Analyt-Rezeptoren gebunden sind, einen Ligand-Konjugat-Referenz-Rezeptor bindet, der so ausgewählt ist, daß er mit dem Ligand-Konjugat bindet, wobei Ligand-Konjugat und Analyt-Rezeptor-Konjugate weiter so ausgewählt werden, daß die Bindungsraten des Ligand-Konjugats an den Referenz-Rezeptor und des Analyt-Rezeptor-Konjugats an die zu bestimmenden Analyten direkt proportional sind zu den Mengen an Referenz-Rezeptor und zu bestimmenden Analyten, die an der Festphase gebunden sind, wobei die Geschwindigkeitskonstanten für die Bindung des Ligand-Konjugats an den Referenz-Rezeptor und für die Bindung der Analyt-Rezeptor-Konjugate an die zu bestimmenden Analyten im wesentlichen gleich sind;

und worin die Entwicklung des Signals im Referenzbereich und den Testbereichen direkt proportional ist zu der Menge an Ligand-Konjugat, das im Referenzbereich gebunden ist, und zu den Mengen an Analyt-Rezeptor-Konjugaten, die in den Testbereichen gebunden sind.

23. Verfahren nach Anspruch 22, worin die zu bestimmenden Analyten FSH und LH oder CKMB und CKMM sind.

24. Verfahren, um innere Standards zur Verwendung in einem Analyt-Rezeptor-Assay für die Bestimmung eines oder mehrerer zu bestimmender Analyten in einer Probe zur Verfügung zu stellen, worin in dem Test ein Analyt-Rezeptor-Konjugat angewendt wird, wobei das Analyt-Rezeptor-Konjugat mit dem zu bestimmenden Analyt vorkomplexiert wird, um einen Komplex des zu bestimmenden Analyten zu bilden, in einer Menge, die direkt proportional ist der Menge an zu bestimmendem Analyt in der Probe, ein Analyt-Rezeptor, der mit dem Komplex des zu bestimmenden Analyten in einer solchen Menge binden kann, die direkt proportional ist zu der Menge des Komplexes des zu bestimmenden Analyten, ein Ligand-Konjugat und ein signalerzeugendes System angewendet wird, wobei das Verfahren umfaßt:

daß man an eine Festphase in einem diskreten Referenzbereich getrennt von einem diskreten Testbereich, in dem der Analyt-Rezeptor gebunden ist, einen Ligand-Konjugat-Referenz-Rezeptor bindet, der so ausgewählt ist, daß er mit einem Ligand-Konjugat bindet;

und wobei die Entwicklung des Signals im Referenzbereich direkt proportional ist zu der Menge des Ligand-Konjugats, das im Referenzbereich gebunden wird, und die Entwicklung des Signals im Testbereich direkt proportional ist zu der Menge des Komplexes des zu bestimmenden Analyten, die im Testbereich gebunden wird, dadurch gekennzeichnet, daß der Referenz-Rezeptor und der Analyt-Rezeptor weiter so ausgewählt sind, daß die Bindungsrate für das Ligand-Konjugat an den Referenz-Rezeptor und die Bindungsrote des Komplexes des zu bestimmenden Analyten an den Analyt-Rezeptor direkt proportional sind zu den Mengen von Ligand-Konjugat und Komplex des zu bestimmenden Analyten.

25. Verfahren nach Anspruch 24, worin ein einzelner zu bestimmender Analyt nachgewiesen wird.

26. Verfahren nach Anspruch 24, worin der Ligand ausgewählt ist aus Hühnereiweißlysozym, Rinderserumalbumin, Meerrettichperoxidase, Bromelin, Fluorescein und Metallkomplexbildnern.

27. Verfahren nach Anspruch 24, worin die Markierungskomponente des Analyt-Rezeptor-Konjugats und des Ligand-Konjugats ausgewählt ist aus einem Enzym, einem Radionuklid, einem fluoreszierenden Mittel, einem phosphoreszierenden Mittel oder einem Polymer, das Farbstoffe oder chemilumineszierende Anteile enthält.

28. Verfahren nach Anspruch 27, worin die Markierung ein Enzym ist.

29. Verfahren nach Anspruch 28, worin das Enzym alkalische Phosphatase oder Meerrettichperoxidase ist.

30. Verfahren nach Anspruch 24, um innere Standards für die Verwendung in einem Analyt-Rezeptor-Assay für die Bestimmung einer Vielzahl von zu bestimmenden Analyten in einer Probe bereitzustellen, wobei in dem Test Analyt-Rezeptor-Konjugate angewendet werden in einer Anzahl, die gleich ist der Zahl der

23

zu bestimmenden Analyten, wobei die Analyt-Rezeptor-Konjugate mit den zu bestimmenden Analyten vorkomplexiert werden, um Komplexe der zu bestimmenden Analyten in solchen Mengen zu bilden, die direkt proportional sind den Mengen an zu bestimmenden Analyten in der Probe, wobei die Analyt-Rezeptoren die Komplexe der zu bestimmenden Analyte binden können in solchen Mengen, die direkt proportional sind zu den Mengen an Komplexen des zu bestimmenden Analyten, ein Ligand-Konjugat und ein signalerzeugendes System angewendet werden, wobei das Verfahren umfaßt:

daß man an eine Festphase in einem diskreten Referenzbereich getrennt von einem diskreten Testbereich, in dem die Analyt-Rezeptoren gebunden sind, einen Ligand-Konjugat-Referenz-Rezeptor bindet, der so ausgewählt ist, daß er das Ligand-Konjugat bindet, wobei der Referenz-Rezeptor und die Analyt-Rezeptoren weiter so ausgewählt sind, daß die Bindungsrate des Ligand-Konjugats an den Referenz-Rezeptor und die Bindungsraten der Komplexe der zu bestimmenden Analyten an die Analyt-Rezeptoren direkt proportional zu den Mengen des Ligand-Konjugats und der Komplexe der zu bestimmenden Analyte sind;

und wobei die Entwicklung des Signals im Referenzbereich direkt proportional zu der Menge an im Referenzbereich gebundenem Ligand-Konjugat ist und die Entwicklung des Signals im Testbereich direkt proportional ist zu der Menge an im Testbereich gebundenen Komplexen der zu bestimmenden Analyte.

**31.** Vorrichtung zur Verwendung in einem Analyt-Rezeptor-Assay für die Bestimmung eines zu bestimmenden Analyten in einer Probe, umfassend:

einen porösen Festphasenträger, umfassend einen diskreten Testbereich, in dem Analyt-Rezeptoren gebunden sind, die den zu bestimmenden Analyt binden können, und mindestens einen diskreten Referenzbereich, in dem ein Referenz-Rezeptor gebunden ist, wobei der Referenz-Rezeptor so ausgewählt ist, daß er mit einem Analyt-Rezeptor-Konjugat, das den zu bestimmenden Analyt binden kann, bindet, und wobei die Entwicklung des Signals im Referenzbereich und im Testbereich direkt proportional ist zu der Menge an im Referenzbereich und im Testbereich gebundenem Analyt-Rezeptor-Konjugat;

dadurch gekennzeichnet, daß der Referenz-Rezeptor weiterhin so ausgewählt ist, daß die Bindungsraten für das Analyt-Rezeptor-Konjugat an den Referenz-Rezeptor und den zu bestimmenden Analyten direkt proportional sind zu den Mengen des Referenz-Rezeptors und des zu bestimmenden Analyten, der an die Festphase gebunden ist, und daß die Geschwindigkeitskonstanten im wesentlichen gleich sind.

**32.** Vorrichtung nach Anspruch 31, worin der Festphasenträger eine poröse Matrix umfaßt, in die in dem diskreten Testbereich Analyt-Rezeptor-gebundene Mikropartikel eingeschlossen sind und im diskreten Referenzbereich Referenz-Rezeptor-gebundene Mikropartikel eingeschlossen sind.

**33.** Vorrichtung nach Anspruch 31 oder 32, umfassend eine Einrichtung, die mit dem porösen Festphasenträger zusammenwirkt, um den Fluß der flüssigen Probe und der Assay-Reagenzien durch den porösen Festphasenträger zu erleichtern.

FIGURE 2.

FIGURE 1

# Figure 3

Figure 4

Binding of Conjugate to Test Zone
50 mIU/mL – Sample LH Concentration

Figure 5

Binding of Conjugate to Reference Zone
2x - Relative Amount of Anti-Alk. Phos.